(19) Europäisches Patentamt
European Patent Office
Office européen des brevets

(11) Veröffentlichungsnummer: **0 215 401 A2**

(12) # EUROPÄISCHE PATENTANMELDUNG

(21) Anmeldenummer: 86112347.9

(22) Anmeldetag: 06.09.86

(51) Int. Cl.⁴: **G 01 N 33/72**

(30) Priorität: 14.09.85 DE 3532868

(43) Veröffentlichungstag der Anmeldung: 25.03.87 Patentblatt 87/13

(84) Benannte Vertragsstaaten: AT BE CH DE FR GB IT LI LU NL SE

(71) Anmelder: **Boehringer Mannheim GmbH, Sandhofer Strasse 116, D-6800 Mannheim 31 (DE)**

(72) Erfinder: **Kerscher, Lorenz, Dr. rer. nat., Paul-Loebe-Strasse 21, D-8122 Penzberg (DE)**
Erfinder: **Rollinger, Wolfgang, Dr. rer. nat., Bärenmühlweg 98, D-8120 Weilheim (DE)**
Erfinder: **Weckerle, Wolfgang, Dr. rer. nat., Auf dem Leimen 12, D-6718 Grünstadt (DE)**
Erfinder: **Klein, Christian, Dr., Blütenstrasse 16, D-8120 Weilheim (DE)**
Erfinder: **Ziegenhorn, Joachim, Dr. rer. nat., Ina-Seidel-Weg 1, D-8130 Starnberg (DE)**

(54) **Verfahren und Reagenz zur Bestimmung von glykosiliertem Hämoglobin sowie hierzu geeignete Verbindungen und Verfahren zu deren Herstellung.**

(57) Beansprucht wird ein Verfahren zur Bestimmung von glykosiliertem Hämoglobin in Blutproben, wobei zunächst durch chemische oder physikalische Behandlung der Blutprobe glykosiliertes und nichtglykosiliertes Hämoglobin aus den Erythrozyten freigesetzt, gegebenenfalls das Hämoglobin in Methämoglobin überführt, danach eine Differenzierung von glykosiliertem und nichtglykosiliertem Hämoglobin mittels Haptoglobin durchgeführt und anschließend der glykosilierte Anteil des Hämoglobins bestimmt wird, dadurch gekennzeichnet, daß die Differenzierung von glykosiliertem und nichtglykosiliertem Hämoglobin mit Haptoglobin in Gegenwart eines geeigneten Puffersystems und/oder einer oder mehrere Substanzen der allgemeinen Formel I

$$A(BR)_n \qquad (I)$$

in der
A ein gesättigter oder ungesättigter, geradkettiger oder verzweigter aliphatischer Kohlenwasserstoffrest, der ein- oder mehrfach durch Heteroatome, durch Oxycycloalkoxyreste oder durch aromatische Reste unterbrochen sein kann, oder ein aromatischer Rest,
R eine Hydroxy-, Hydroxyalkyl-, Hydroxyalkoxygruppe, eine Aminogruppe, eine reaktive Gruppe X, einen Säurerest bzw. dessen Salze oder einen ein- oder mehrfach durch Säurereste

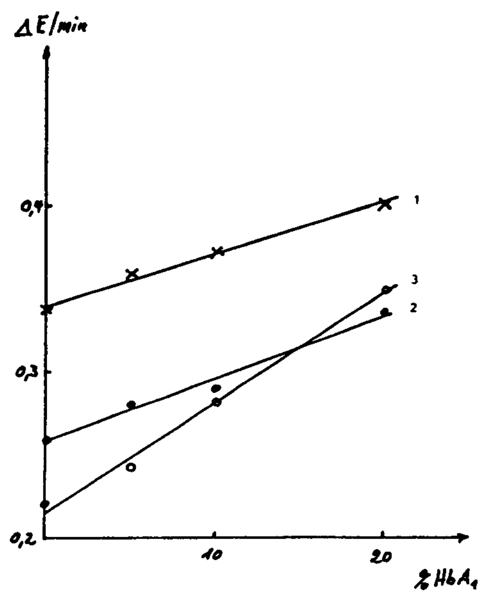


EP 0 215 401 A2

bzw. deren Salze oder durch reaktive Gruppen X substituierten Alkyl-, Alkoxy-, Hydroxyalkyl- oder Hydroxyalkoxyrest, die durch Heteroatome unterbrochen sein können,
B eine Einfachbindung oder einen unsubstituierten oder einen ein- oder mehrfach durch weitere Reste R substituierten aromatischen Rest, und
n eine ganze Zahl von 2 bis 20
bedeuten,

wobei sowohl die verschiedenen Reste B als auch R innerhalb einer Substanz auch unterschiedlich sein können, erfolgt, sowie hierfür geeignete Reagentien.

BOEHRINGER MANNHEIM GMBH



# Verfahren und Reagenz zur Bestimmung von glykosiliertem Hämoglobin sowie hierzu geeignete Verbindungen und Verfahren zu deren Herstellung

Die vorliegende Erfindung betrifft ein Verfahren zur Bestimmung von glykosiliertem Hämoglobin in Blutproben, ein Reagenz zu seiner Durchführung sowie hierzu geeignete Verbindungen und Verfahren zu deren Herstellung.

Glykosiliertes Hämoglobin entsteht durch nichtenzymatische Glykosilierung des Hämoglobins. Normalerweise beträgt die Konzentration an glykosiliertem Hämoglobin im Blut etwa 5 %, bezogen auf das Gesamthämoglobin. Bei Diabetikern ist diese Konzentration auf das Zwei- bis Vierfache erhöht.

Die Bestimmung des glykosilierten Anteils am Gesamthämoglobin hat in den letzten Jahren Bedeutung bei der Diabetesdiagnostik erlangt (vergleiche L. Jovanovic und C. M. Peterson, Am. J. Med. 70, (1981), Seite 331 - 338). Die Reaktion zwischen einzelnen Hämoglobinmolekülen und Glukose ergibt ein stabiles Reaktionsprodukt, das während der gesamten Lebenszeit der Erythrozyten, also ca. 100 - 200 Tage, erhalten bleibt. Kurzzeitige Schwankungen des Blutzuckergehaltes beeinflussen die Konzentration an glykosiliertem Hämoglobin nicht entscheidend. Die Konzentration an glykosiliertem Hämoglobin spiegelt daher relativ genau die durchschnittliche Glukosekonzentration im Blut eines Patienten über einen langen Zeitraum hinweg wieder.

Es sind mehrere Verfahren zur Bestimmung des glykosilierten Anteils am Gesamthämoglobin bekannt. In klinischen Labors am weitesten verbreitet sind chromatographische Trennverfahren. Hierbei wird der glykosilierte Anteil des Hämoglobins vom nichtglykosilierten mit Hilfe einer Chromatographiesäule, einer Mikrosäule oder auch mit Hilfe der HPLC-Methode (HPLC = high pressure liquid chromatography)

getrennt, wobei die Säulen mit einem Ionenaustauscher, beispielsweise mit Bio Rex 70 gefüllt sind. Alle diese Methoden sind jedoch sehr empfindlich gegen Änderungen des pH-Wertes, der Temperatur und der Ionenkonzentrationen. Die Trennverfahren müssen daher sehr vorsichtig durchgeführt werden, um optimale Resultate zu erhalten (vergleiche a.a.O. Seite 332).

Aus der deutschen Offenlegungsschrift 29 50 457 ist ein Verfahren zur Bestimmung des glykosilierten Hämoglobins in Blutproben bekannt, bei dem die Veränderung der spektroskopischen Eigenschaften einer Blutprobe bei Zugabe eines allosterischen Effektors für die Bestimmung des glykosilierten Hämoglobins ausgenutzt wird. Beeinflußt wird hierbei lediglich der nichtglykosilierte Hauptanteil des Hämoglobins. Die gemessenen Extinktionsdifferenzen sind im relevanten Bereich sehr klein. Sie werden außerdem noch geringer, wenn der glykosilierte Anteil am Gesamthämoglobin zunimmt.

Ein Verfahren zur Bestimmung von glykosiliertem Hämoglobin aufgrund dessen verstärkter Bindungsfähigkeit an Haptoglobin wird in der deutschen Offenlegungsschrift 31 41 146 beschrieben. Der Unterschied im Bindungsverhalten von glykosiliertem und nichtglykosiliertem Hämoglobin gegenüber Haptoglobin wird durch Zusatz von 2,3-Diphosphoglycerat, Inosithexaphosphat oder Mellitsäure verstärkt. Er wird jedoch in keinem Fall so ausgeprägt, daß ausschließlich das glykosilierte Hämoglobin an Haptoglobin gebunden wird. Eine einfache photometrische Bestimmung des Haptoglobin-Hämoglobin-Komplexes ist entsprechend der deutschen Offenlegungsschrift 33 14 308 durch Messung der Pseudoperoxidaseaktivität dieses Komplexes möglich, wobei die Aktivität des freien, nichtglykosilierten Hämoglobins durch Detergenzzusatz und Wahl geeigneter chromogener Substrate unterdrückt wird.

Mit Kenntnis des Standes der Technik gelangt man so zu einem homogenen Test auf glykosiliertes Hämoglobin, welcher die folgenden drei Inkubationsschritte umfaßt:

1. Hämolyse und Überführung der Hämgruppen von sowohl glykosiliertem als auch nichtglykosiliertem Hämoglobin in eine einheitlich ligandierte Form einer einzigen Oxidationsstufe, bevorzugt Nitrosyl-Hämoglobin,

2. Reaktion mit Haptoglobin,

3. Bestimmung des glykosilierten Hämoglobins, z. B. durch Denaturierung des freien Hämoglobins und kinetischer, photometrischer Messung der Peroxidaseaktivität der Hämoglobin-Haptoglobinkomplexe.

Derzeitige Analysenautomaten sind in der Regel nur auf die Ausführung von einem oder höchstens zwei Inkubationsschritten ausgelegt. Deshalb ist der vorgenannte Reaktionsablauf in der klinischen Praxis nicht vollständig automatisierbar. Andererseits hängt bei manueller Durchführung eines derartigen, mindestens vier Pipettiervorgänge umfassenden Reaktionsablaufes die Präzision der Bestimmung in großem Maße vom Geschick des Ausführenden ab. Außerdem ergeben die bisher bekannten Verfahren zur Bestimmung von glykosiliertem Hämoglobin auch für nichtglykosiliertes Hämoglobin ein Meßsignal, welches die Empfindlichkeit des Tests herabsetzt und zur Erstellung einer Eichgeraden zwingt.

Aufgabe der Erfindung war es daher, die von der Präzision und der Spezifität der Indikatorreaktion abhängige Empfindlichkeit der Bestimmung von glykosiliertem Hämoglobin zu verbessern und Möglichkeiten für eine vollautomatische Testdurchführung zu schaffen.

Gelöst wird diese Aufgabe durch das erfindungsgemäße Verfahren, bei dem nach einer chemischen oder physikalischen Behandlung der Blutprobe zur Freisetzung des Hämoglobins aus den Erythrozyten eine Differenzierung des glykosilierten und nichtglykosilierten Hämoglobinanteils mit Hilfe von Haptoglobin in Gegenwart von Verbindungen erfolgt, die den Unterschied im Bindungsverhalten von glykosiliertem und nichtglykosiliertem Hämoglobin gegenüber Haptoglobin im Vergleich zu bisher bekannten Substanzen wesentlich erhöhen und entweder die Reaktion zwischen Hämoglobin und

Haptoglobin kinetisch verfolgt oder der an Haptoglobin gebundene bzw. der von Haptoglobin nichtgebundene Teil des Hämoglobins gemessen wird.

Gegenstand der vorliegenden Erfindung ist somit ein Verfahren zur Bestimmung von glykosiliertem Hämoglobin in Blutproben, wobei zunächst durch chemische oder physikalische Behandlung der Blutprobe glykosiliertes und nichtglykosiliertes Hämoglobin aus den Erythrozyten freigesetzt, danach eine Differenzierung von glykosiliertem und nichtglykosiliertem Hämoglobin mittels Haptoglobin durchgeführt und anschließend der glykosilierte Anteil des Hämoglobins bestimmt wird, dadurch gekennzeichnet, daß die Differenzierung von glykosiliertem und nichtglykosiliertem Hämoglobin mit Haptoglobin in Gegenwart einer oder mehrerer Substanzen der allgemeinen Formel I

$$A(BR)_n \qquad (I)$$

in der

A ein gesättigter oder ungesättigter, geradkettiger oder verzweigter aliphatischer Kohlenwasserstoffrest, der ein- oder mehrfach durch Heteroatome, durch Oxycycloalkoxyreste oder durch aromatische Reste unterbrochen sein kann, oder ein aromatischer Rest,

R eine Hydroxy-, Hydroxyalkyl-, Hydroxyalkoxygruppe, eine Aminogruppe, eine reaktive Gruppe X, einen Säurerest bzw. dessen Salze oder einen ein- oder mehrfach durch Säurereste bzw. deren Salze oder durch reaktive Gruppen X substituierten Alkyl-, Alkoxy-, Hydroxyalkyl- oder Hydroxyalkoxyrest, die durch Heteroatome unterbrochen sein können,

B eine Einfachbindung oder einen unsubstituierten oder einen ein- oder mehrfach durch weitere Reste R substituierten aromatischen Rest und

n eine ganze Zahl von 2 bis 20

bedeuten,

wobei sowohl die verschiedenen Reste B als auch R innerhalb einer Substanz auch unterschiedlich sein können,

erfolgt.

Ein gesättigter oder ungesättigter, geradkettiger oder verzweigter aliphatischer Kohlenwasserstoffrest in der Definition von A besitzt vorzugsweise zwei bis 30, besonders bevorzugt 2 bis 10 Kohlenstoffatome.

Heteroatome, die aliphatischen Kohlenwasserstoff unterbrechen können, sind vorzugsweise Sauerstoff und Schwefel.

Oxycycloalkoxyreste bestehen aus 5 bis 8 Kohlenstoffatomen. Bevorzugt sind Reste wie z. B. Oxycyclopentoxy oder Oxycyclohexoxy. Besonders bevorzugt ist der Oxycyclohexoxyrest. Die Oxycycloalkoxyreste können an jedem Ring-C-Atom durch weitere Reste B-R substituiert sein.

Aromatische Reste in der Definition von A und B können z. B. Benzol oder Heteroaromaten wie Pyridin, Furan oder Pyrrol sein.

Unter Alkyl, Alkoxy, Hydroxyalkyl bzw. Hydroxyalkoxy in der Definition von R sind Reste aus 1 - 10, bevorzugt 1 - 6 Kohlenstoffatomen zu verstehen. Besonders bevorzugt sind Reste aus 2 bis 5 Kohlenstoffatomen. Meist verwendet werden Reste mit drei oder vier Kohlenstoffatomen.

Unter einer reaktiven Gruppe X werden Gruppen verstanden, die mit Aminogruppen eine kovalente Bindung eingehen können, und zwar ohne zusätzliche Hilfsmittel, wie z. B. Dicyclohexylcarbodiimid oder erhöhte Temperatur (über 40° C). Bevorzugte Gruppen X sind z. B. der Aldehydrest oder dessen Bisulfitaddukt oder der Thiocyanat- oder Isothiocyanatrest. Besonders bevorzugt ist der Aldehydrest.

Säurereste im Sinne der vorliegenden Erfindung sind vorzugsweise -COOH, $-SO_3H$, $-OSO_3H$, $-PO_3H_2$, $-OPO_3H_2$, $-P_2O_6H_3$, $-O-P_2O_6H_3$. Als Salze dieser Säurereste sind die Alkali-, Erdalkali- und Ammoniumsalze bevorzugt. Unter Alkalisalzen sind vor allem Lithium-, Natrium- und Kaliumsalze zu verstehen. Erdalkalisalze sind vor allem Magnesium-, Calcium- oder Bariumsalze. Ammoniumsalze können unsubstituierte Ammoniumionen enthalten oder solche, die durch Alkylgruppen mit 1 - 5 Kohlenstoffatomen, bevorzugt Methyl- oder Ethylresten, oder auch durch Aralkylgruppen, wie Benzylreste, substituiert sind. Die Substituenten können hierbei gleich oder verschieden sein.

Substanzen der allgemeinen Formel I können der Blutprobe vor, aber auch gleichzeitig mit dem Haptoglobin zugegeben werden und haben den überraschenden Effekt, daß die Bindung von nichtglykosiliertem Hämoglobin an Haptoglobin vollständig unterdrückt wird, so daß der Test spezifisch für glykosiliertes Hämoglobin wird. Es ist möglich, aber in Gegenwart dieser Substanzen nicht mehr notwendig, die Hämgruppen schon vorher in eine einheitliche Form zu überführen, so daß auch ein voll automatisierbarer, nur zwei Inkubationsschritte umfassender Versuchsablauf realisierbar wird.

Das Verfahren zur Bestimmung des glykosilierten Anteils am Gesamthämoglobin wird zweckmäßigerweise derart durchgeführt, daß man zunächst nach üblichen Methoden aus den Erythrozyten das glykosilierte und das nichtglykosilierte Hämoglobin freisetzt. Gleichzeitig mit dem Hämolysereagenz kann eine oder mehrere Verbindungen der allgemeinen Formel I zugegeben werden. Im Falle der physikalischen Behandlung von Blutproben zur Freisetzung glykosilierten und nichtglykosilierten Hämoglobins aus den Erythrozyten kann die Probe bereits vor oder auch während dieses Vorganges mit Verbindungen der allgemeinen Formel I versetzt werden. Der hämolysierten Blutprobe wird dann eine Haptoglobin enthaltende Lösung zugegeben. Unter diesen Bedingungen bindet der glykosilierte Anteil des Hämoglobins an Haptoglobin. Die Bestimmung der Haptoglobin-Hämoglobinkomplexe kann dann anschließend z. B. in bekannter Weise durch Messung der Peroxidaseaktivität nach Denaturierung des freien, nichtglykosilierten Hämoglobins erfolgen.

Das erfindungsgemäße Verfahren wird zweckmäßig in Gegenwart eines geeigneten Puffersystems durchgeführt. Als geeignetes Puffersystem kann jeder im pH-Bereich von etwa 4,0 bis etwa 8,5, vorzugsweise von etwa 6,0 bis etwa 7,0 wirksame Puffer verwendet werden. Besonders bewährt haben sich Phosphat-, Morpholinoethansulfonsäure (MES)- und Bis-Tris-Puffer.

In einer speziellen Ausführungsform des erfindungsgemäßen Verfahrens wird die Differenzierung von glykosiliertem und nichtglykosiliertem Hämoglobin mittels Haptoglobin in Gegenwart von Substanzen durchgeführt, welche ionische Bindungen ausbilden können. Dies sind Verbindungen der allgemeinen Formel II

$$A'(R')_n \qquad (II)$$

in der

A' ein gesättigter oder ungesättigter, geradkettiger oder verzweigter aliphatischer Kohlenwasserstoffrest, der ein- oder mehrfach durch Heteroatome oder Oxycycloalkoxyreste unterbrochen sein kann, oder ein aromatischer Rest,

R' eine Hydroxy- oder Aminogruppe, ein Säurerest bzw. dessen Salze, einen ein- oder mehrfach durch Säurereste bzw. deren Salze substituierter Alkyl-, Alkoxy-, Hydroxyalkyl- oder Hydroxyalkoxyrest, wobei die Reste R' innerhalb einer Substanz gleich oder verschieden sein können, und

n eine ganze Zahl von 2 bis 20

bedeuten.

Für die in den Definitionen von A' und R' aufgeführten Reste gilt das oben im Zusammenhang mit A und R Gesagte.

Unter den in der Definition von R' genannten Säureresten sind bei den Verbindungen der allgemeinen Formel II vor allem jene bevorzugt, welche mehrwertige Anionen bilden können. Ganz besonders bevorzugt sind deshalb $-OPO_3H_2$ und $-PO_3H_2$.

Bevorzugte Verbindung der allgemeinen Formel II sind z. B.

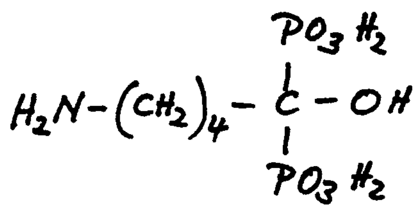

oder deren Alkali-, Erdalkali- oder Ammoniumsalze.

Ist A' in der Definition der allgemeinen Formel II ein gesättigter, geradkettiger aliphatischer Kohlenwasserstoffrest, sind Verbindungen der allgemeinen Formel II a

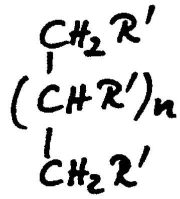

(II a)

in der

R' und n die oben angegebene Bedeutung haben, für das erfindungsgemäße Verfahren bevorzugt.

Besonders bevorzugte Verbindungen der allgemeinen Formel II a sind z. B.

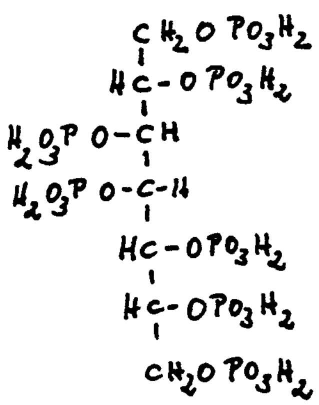

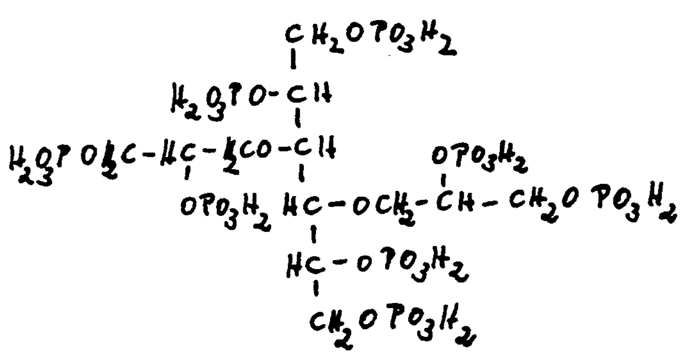

oder deren Alkali-, Erdalkali- oder Ammoniumsalze. Diese Verbindungen sind neu.

Ist A' in der Definition der allgemeinen Formel II ein durch ein Oxycycloalkoxyrest unterbrochener aliphatischer Kohlenwasserstoffrest, so sind für das erfindungsgemäße Verfahren Verbindungen der allgemeinen Formel II b

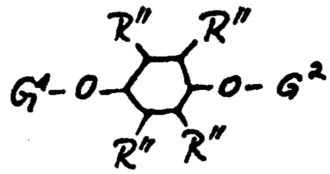

(II b)

in der

R'' eine Hydroxygruppe, eine Aminogruppe, ein Säurerest bzw. dessen Salze, wobei die Reste R'' innerhalb einer Substanz gleich oder verschieden sein können, und

$G^1$ und $G^2$, die gleich oder verschieden sein können, einen Alkyl- oder Hydroxyalkylrest, der ein- oder mehrfach durch Reste R'' substituiert ist,

bedeuten, bevorzugt.

Für den Säurerest bzw. dessen Salze in der Definition von R'' gilt das oben für die entsprechenden Reste bei der Definition von R Gesagte.

Mit Alkyl- oder Hydroxyalkylresten in der Definition von $G^1$ und $G^2$ sind Reste aus 1 - 6 Kohlenstoffatomen, bevorzugt 1 - 4 Kohlenstoffatomen gemeint. Meist verwendet werden Reste aus 3 oder 4 Kohlenstoff-Atomen.

Verbindungen der allgemeinen Formel II b sind neu. Besonders bevorzugte Vertreter sind z. B.

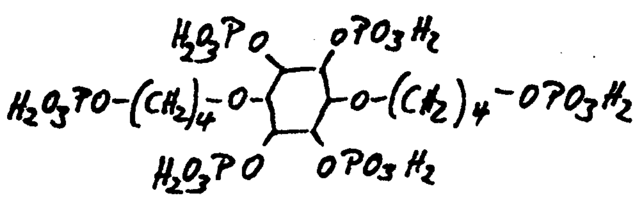

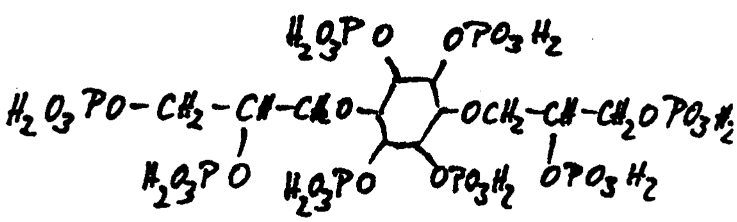

oder deren Alkali-, Erdalkali- oder Ammoniumsalze.

Ist A' in der Definition der allgemeinen Formel II ein aromatischer Rest, dann sind für das erfindungsgemäße Verfahren Verbindungen der allgemeinen Formel II c

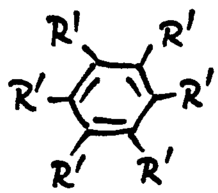

(II c)

in der

R' die in Formel II angegebene Bedeutung hat, bevorzugt.

Verbindungen der allgemeinen Formel II c sind neu. Besonders bevorzugte Vertreter sind z. B.

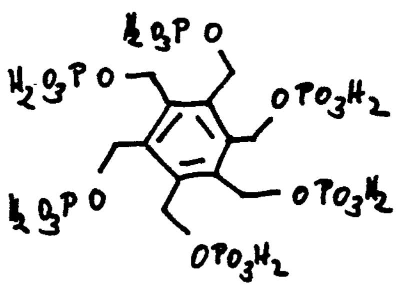

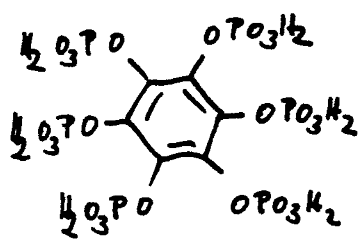

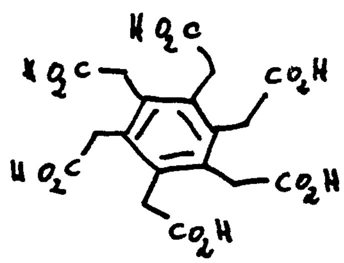

oder deren Alkali-, Erdalkali- oder Ammoniumsalze.

Die Herstellung von Verbindungen der allgemeinen Formeln II, II a, II b oder II c erfolgt nach an sich bekannten Methoden. Ausgehend von entsprechenden Polyolen werden $OPO_3H_2$- oder $OP_2O_6H_3$-Gruppen oder deren Alkali-, Erdalkali- oder Ammoniumsalze mit geeigneten Phosphorylierungsmitteln [vgl. z. B. Adv. Org. Chem. 3, 75 - 157 (1963) oder Angew. Chem. 76, 704 - 712 (1964)], wie Polyphosphorsäure, Halogenphosphorsäureverbindungen oder Amidophosphorsäurederivaten eingeführt.

Die Reaktion wird zweckmäßigerweise ohne Lösungsmittel durchgeführt oder in einem Lösungsmittel wie Dichlormethan, Chloroform, Formamid, Dimethylformamid, Benzol, Tetrachlorkohlenstoff, Aceton, Acetonitril, Pyridin, Lutidin oder Collidin.

Phosphorylierungen finden mit oder ohne Zusatz von Hilfsreagentien, wie Silbersalze, Ammoniak, gegebenenfalls substituierte Amine, wie Triethylamin, Cyclohexylamin, Piperidine, Piperazine oder Pyridin statt.

Die Isolierung der Produkte erfolgt durch Fällung als Salz, bevorzugt als Bariumsalz oder durch Ionenaustauschchromatographie.

Die erhaltenen Verbindungen können durch Elementaranalyse, $^{1}H$-, $^{13}C$- und $^{31}P$-NMR-Spektroskopie sowie durch die elektrophoretische Mobilität, $M_{rel}$, bezogen auf Pyrophosphat ($M_{rel}$ = 1) auf cellulosebeschichteten Glasplatten mit einem geeigneten Puffersystem charakterisiert werden.

Sollen nur partiell phosphorylierte Produkte erhalten werden, müssen die entsprechenden Hydroxygruppen vorübergehend, während der Phosphorylierungsreaktion, durch geeignete Schutzgruppen geschützt werden.

Geeignete Schutzgruppen sind insbesondere die Acetyl-, die Benzoyl-, die Benzyl-, die Isopyropyliden-, die Cyclohexyliden- oder die Benzyliden-Gruppe. Nach erfolgter Phosphorylierung werden die Schutzgruppen in bekannter Weise wieder abgespalten und die geschützten Alkoholfunktionen wieder freigesetzt.

Phosphonsäurederivate werden nach üblichen Methoden hergestellt. Vor allem geminale Diphosphonsäureverbindungen werden bevorzugt durch nachfolgende Umsetzung entsprechender Carbonylverbindungen mit phosphoriger Säure und Phosphortrichlorid in hochsiedenden Lösungsmitteln, wie z. B. Chlorbenzol und abschließende saure Hydrolyse der entstandenen Zwischenverbindungen erhalten.

Die Reaktionstemperaturen sollten vorteilhafterweise etwa 70 - 140° C betragen. Besonders bevorzugt sind Temperaturen um 100° C.

Bei solchen Temperaturen betragen die Reaktionszeiten einige Stunden, in den meisten Fällen 2 - 7 Stunden.

Die Isolierung der Verbindungen erfolgt vorteilhafterweise durch Ausfällung als Salz in organischen, mit Wasser mischbaren Lösungsmitteln, wie z. B. Methanol oder Ethanol oder durch Ionenaustauschchromatographie.

Die Einführung von Sulfonsäuregruppen in den aliphatischen wie in den aromatischen Teil erfolgt durch übliche Sulfonierungsreaktionen. Als besonders günstig hat sich die Sulfonierung mit Chlorsulfonsäure erwiesen. Die aromatische Substitution führt bevorzugt bei erhöhter Temperatur, vorzugsweise 40 - 80° C, in guter Ausbeute zum Ziel. Überführung der aliphatischen Alkoholfunktionen der Ausgangsverbindungen in $OSO_3H$-Gruppen gelingt mit Chlorsulfonsäure dagegen bereits bei niedrigen Temperaturen, vorzugsweise bei 0 - 20° C.

Zur Herstellung von Carbonsäurederivaten können Carboxygruppen in Polyole nach bekannten Methoden, z. B. durch Überführung primärer oder sekundärer Alkoholfunktionen in gute Abgangsgruppen, Substitution dieser Abgangsgruppen durch Cyanid und abschließende Hydrolyse des Nitrils eingebracht werden.

Als gute Abgangsgruppen gelten insbesondere z. B. die Acetyl-, die Benzoyl-, Toluolsulfonyl-, p-Brombenzolsulfonyl-, Methansulfonyl- oder Trifluormethansulfonylgruppe. Sie können in einfacher Weise durch Reaktion des Polyols, z. B. mit dem entsprechenden Säurechlorid oder Säureanhydrid eingeführt werden.

Zur Substitutionsreaktion mit Cyanid können Alkali-, Erdalkali- oder Ammoniumcyanide verwendet werden. Je nach Lösungsmittel und gewähltem Cyanid kann die Reaktion unter homogenen oder heterogenen Bedingungen, z. B. unter Phasentransferkatalyse stattfinden.

Hydrolyse des als Zwischenprodukt entstehenden Nitrils ist sowohl sauer als auch basisch möglich. Bevorzugt werden basische Bedingungen gewählt.

Die Isolierung der Produkte erfolgt durch Fällung als Salz, bevorzugt als Bariumsalz oder durch Ionenaustauschchromatographie.

Sollen nur partiell carboxylierte Produkte erhalten werden, müssen die entsprechenden Hydroxygruppen vorübergehend, während der Überführung von OH-Gruppen in gute Abgangsgruppen, durch geeignete Schutzgruppen geschützt werden. Als geeignete Schutzgruppen gelten insbesondere die Benzyl-, die Isopropyliden-, die Cyclohexyliden- oder die Benzyliden-Gruppe. Nach erfolgter Überführung der zu substituierenden Hydroxylgruppen in gute Abgangsgruppen können die Schutzgruppen in geeigneter Weise wieder abgespalten werden.

In einer bevorzugten Ausführungsform des erfindungsgemäßen Verfahrens wird die Differenzierung von glykosiliertem und nichtglykosiliertem Hämoglobin mittels Haptoglobin in Gegenwart von Substanzen durchgeführt, die sowohl ionische als auch kovalente Bindungen ausbilden können. Dies sind Verbindungen der allgemeinen Formel III

$$X^1\text{-}B^1\text{-}A''\text{-}B^2\text{-}X^2 \qquad \text{(III)}$$

in der

A'' dieselbe Bedeutung wie A in Formel I hat, wobei A'' ein- oder mehrfach durch Reste R substituiert sein kann, wobei R die in Formel I angegebene Bedeutung hat,

$B^1$ und $B^2$, die gleich oder verschieden sein können, dieselbe Bedeutung wie B in Formel I haben, und

$X^1$ und $X^2$, die gleich oder verschieden sein können, jeweils eine reaktive Gruppe

vorstellen.

Für die reaktiven Gruppen $X^1$ und $X^2$ gelten die Ausführungen, die oben für die reaktive Gruppe X gemacht worden sind, in entsprechender Weise.

Eine bevorzugte Verbindung der allgemeinen Formel III ist z. B.

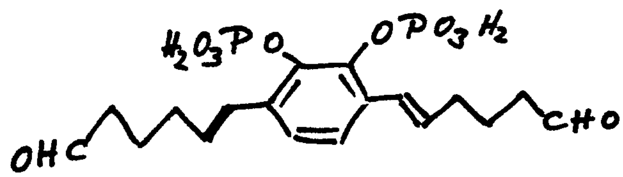

oder deren Alkali-, Erdalkali- oder Ammoniumsalze.

Im Fall, daß A'' in der Definition der allgemeinen Formel III ein gesättigter, durch Heteroatome unterbrochener, aliphatischer Kohlenwasserstoffrest und $B^1$ und $B^2$ jeweils eine Einfachbindung darstellen, sind für das erfindungsgemäße Verfahren Verbindungen der allgemeinen Formel III a

$$X^1\text{-}(CH_2)_p\text{-}O\text{-}(CHR''')_m\text{-}O\text{-}(CH_2)_x\text{-}X^2 \quad \text{(III a)}$$

in der

R''' Wasserstoff, eine Hydroxygruppe, einen Säurerest bzw. dessen Salze oder einen gegebenenfalls ein- oder mehrfach durch Säurereste bzw. deren Salze substituierter Alkyl-, Alkoxy-, Hydroxyalkyl- oder Hydroxyalkoxyrest,

$X^1$ und $X^2$ eine reaktive Gruppe und

p, m, x jeweils eine ganze Zahl von 1 - 10

bedeuten, wobei bei Vorkommen mehrerer Reste R''' in einer Verbindung diese Reste gleich oder verschieden sein können, bevorzugt.

Für die Definitionen von R''' gilt das oben im Zusammenhang mit R Gesagte.

Für die reaktive Gruppen $X^1$ und $X^2$ gelten die Ausführungen, die oben für die reaktive Gruppe X gemacht worden sind, in entsprechender Weise.

Verbindungen der allgemeinen Formel III a, in der mindestens ein Rest R''' einen Säurerest bzw. dessen Salze oder einen ein- oder mehrfach durch Säurereste bzw. dessen Salze substituierten Alkyl-, Alkoxy-, Hydroxyalkyl- oder Hydroxyalkoxyrest bedeutet, sind neu.

Besonders bevorzugte Vertreter sind z. B.

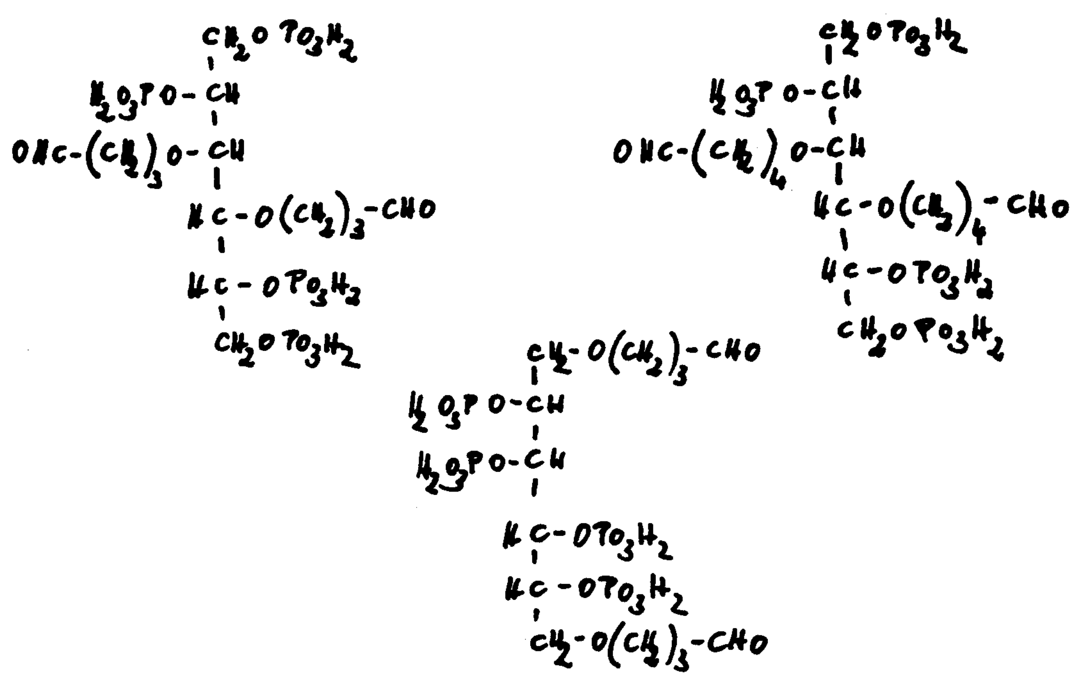

oder deren Alkali-, Erdalkali- oder Ammoniumsalze.

Sind A'' in der Definition der allgemeinen Formel III ein aliphatischer Kohlenwasserstoffrest, der durch einen Oxycycloalkoxyrest unterbrochen ist, und $B^1$ und $B^2$ jeweils eine Einfachbindung, dann werden für das erfindungsgemäße Verfahren Verbindungen der allgemeinen Formel III b

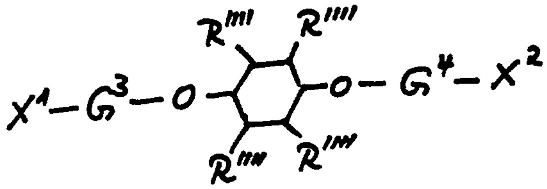

(III b)

in der

R'''' Wasserstoff, ein Säurerest bzw. dessen Salze, wobei die verschiedenen Reste R'''' innerhalb einer Verbindung gleich oder verschieden sein können,

$G^3$ und $G^4$, die gleich oder verschieden sein können, jeweils eine Kohlenwasserstoffkette und

$X^1$ und $X^2$, die gleich oder verschieden sein können, eine reaktive Gruppe

bedeuten, bevorzugt.

Für den Säurerest bzw. dessen Salze in der Definition von R'''' gilt das oben für die entsprechenden Reste bei der Definition von R Gesagte.

Die Kohlenwasserstoffkette in der Definition von $G^3$ und $G^4$ besteht aus 1 bis 7, vorzugsweise 2 bis 5 Kohlenstoffatomen.

Für die reaktiven Gruppen $X^1$ und $X^2$ gelten die Ausführungen, die oben für die reaktive Gruppe X gemacht worden sind, in entsprechender Weise.

Verbindungen der allgemeinen Formel III b sind neu. Besonders bevorzugte Vertreter sind z. B.

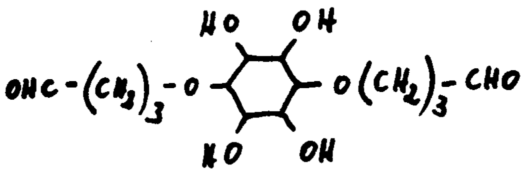 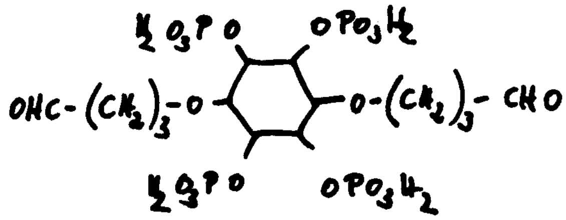

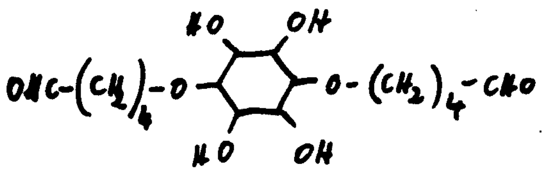 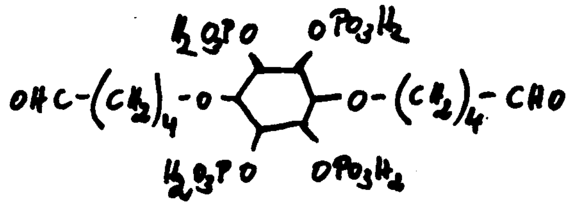

oder deren Alkali-, Erdalkali- oder Ammoniumsalze.

Die Herstellung von Verbindungen der allgemeinen Formel III a oder III b erfolgt nach an sich bekannten Methoden, welche prinzipiell denen für die Herstellung von Verbindungen der allgemeinen Formel II a, II b und II c entsprechen.

Ausgangsstoffe sind jeweils Polyole deren Hydroxygruppen analog der für Verbindungen der allgemeinen Formeln II, II a, II b und II c geschilderten Methoden in Säuregruppen überführt werden können. Die Einführung reaktiver Gruppen $X^1$ und $X^2$ ist abhängig von der Art dieser Gruppen.

Aldehydfunktionen werden üblicherweise durch Oxidation primärer Alkoholfunktionen gebildet. Als Oxidationsmittel werden Mangandioxid oder Manganatsalze, wie z. B. Bariummanganat verwendet.

Um unter diesen Bedingungen eine selektive Oxidation bestimmter OH-Gruppen eines Polyols durchführen zu können, müssen vorher diejenigen Hydroxygruppen, die nicht angegriffen werden sollen, geschützt werden. Dies geschieht durch geeignete Schutzgruppen, wie

z. B. die Acetyl-, Benzoyl-, Benzyl-, Isopropyliden-, Cyclohexyliden- oder Benzyliden-Gruppe. Nach erfolgter Einführung der Aldehydfunktionen werden die Schutzgruppen in bekannter Weise wieder abgespalten und die Alkoholfunktionen wieder freigesetzt. Anschließend können diese dann in Säuregruppen überführt werden.

Bisulfitaddukte von Aldehyden sind leicht durch Reaktion mit Natriumhydrogensulfit herstellbar, indem Aldehyde z. B. in Bisulfitlauge gelöst werden. Die Reaktionsmischung kann entweder direkt, ohne Isolierung des Endprodukts, oder auch erst nach Reinigung und Isolierung des Addukts für das erfindungsgemäße Verfahren zur Bestimmung von glykosiliertem Hämoglobin eingesetzt werden.

Isothiocyanate werden vorteilhafterweise aus primären Aminen und Schwefelkohlenstoff in Gegenwart einer starken Base hergestellt. Unter starken Basen werden hierbei vor allem Alkali- und Erdalkalihydroxide verstanden. Das sich bildende Salz der N-Alkyl-dithio-carbamidsäure reagiert anschließend z. B. mit Chlorameisensäureestern zu den gewünschten Isothiocyanaten.

Besonders bevorzugte Verbindungen der allgemeinen Formel III sind die Benzylverbindungen der allgemeinen Formel IV

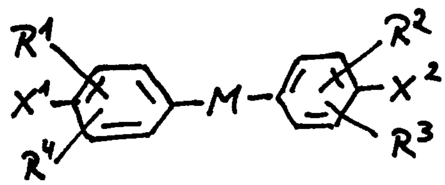

IV

in der

M eine Einfachbindung oder eine gesättigte oder ungesättigte Kohlenwasserstoffkette, die gegebenenfalls ein- oder mehrfach durch Säurereste bzw. deren Salze substituiert ist, bedeutet,

$R^1$, $R^2$, $R^3$ und $R^4$, die gleich oder verschieden sein können, die Bedeutung von R''' in Formel III a haben und

$X^1$ und $X^2$, die gleich oder verschieden sein können, eine reaktive Gruppe vorstellen.

Unter der gesättigten oder ungesättigten Kohlenwasserstoffkette in der Definition von M wird ein Rest aus 1 bis 8, bevorzugt 1 bis 4 Kohlenstoffatomen verstanden.

Für die reaktiven Gruppen $X^1$ und $X^2$ gelten die Ausführungen, die oben für die reaktive GruppeX gemacht worden sind, in entsprechender Weise.

Verbindungen der allgemeinen Formel IV, in der M eine durch Säuregruppen bzw. deren Salze ein- oder mehrfach substituierter Kohlenwasserstoffrest ist, sind neu.

Besonders bevorzugte Vertreter sind z. B.

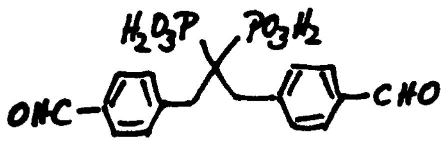

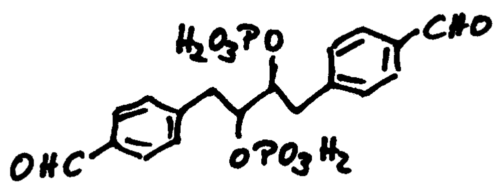

oder deren Alkali-, Erdalkali- oder Ammoniumsalze.

Die Herstellung der Verbindungen der allgemeinen Formel IV erfolgt nach an sich bekannten Methoden.

Besonders bevorzugte Verbindungen der allgemeinen Formel IV sind Verbindungen der allgemeinen Formel V

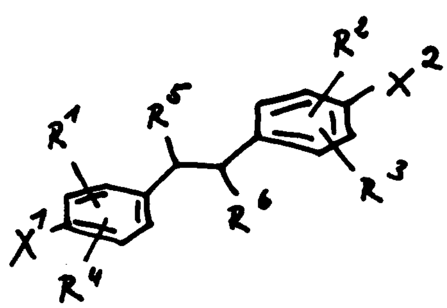

(V)

in der

$R^1$, $R^2$, $R^3$, $R^4$, $X^1$ und $X^2$ die in Formel IV angegebene Bedeutung haben und

$R^5$ und $R^6$, die gleich oder verschieden sein können, einen Säurerest bzw. dessen Salze vorstellen.

Als Säurerest in den Definitionen von $R^5$ und $R^6$ sind $-OPO_3H_2$, $-OP_2O_6H_3$ oder $-OSO_3H$ bevorzugt.

Als Verbindungen, welche das erfindungsgemäße Verfahren zur Bestimmung glykosilierten Hämoglobins besonders günstig beeinflussen, sind 1,2-Bis(4-formyl-2-sulfonylphenyl)ethan und 1,2-Bis(4-formylphenyl)ethandiol-1,2-diphosphat zu nennen.

Dibenzylverbindungen der allgemeinen Formel V sind neu. Sie können nach an sich bekannten Methoden hergestellt werden. Das 1,2-Bis(4-formylphenyl)ethan-gerüst erhält man z. B. durch Wittig-Reaktion eines p-Alkoxycarbonylbenzyltriphenylphosphoniumbromids mit einem p-Alkoxycarbonylbenzaldehyd. Unter Alkoxyresten werden in diesem Zusammenhang Gruppen aus 1 - 5 Kohlenstoffatomen verstanden. Besonders bevorzugt sind der Methoxy- oder Ethoxy-Rest. Das gebildete Stilben wird z. B. über Palladium auf Kohle hydriert. Die beiden Carbonsäureester-Funktionen wandelt man anschließend in bekannter Weise, z. B. durch Reduktion mit Lithiumaluminiumhydrid zum Benzylalkohol und anschließende Braunsteinoxidation zum Aldehyd um.

Sulfonsäuregruppen werden durch übliche Sulfonierungsreaktionen eingeführt. Als besonders günstig hat sich die Sulfonierung mit Chlorsulfonsäure erwiesen, welche bei erhöhter Temperatur, vorzugsweise 40 - 80° C, in guter Ausbeute zum Ziel führt.

Carboxy- und Carboxymethylenoxygruppen können durch Wahl entsprechend geeigneter, literaturbekannter Ausgangsverbindungen eingeführt werden.

Phosphatgruppen an der Ethylengruppierung werden eingeführt, indem man das entsprechende Stilben zunächst nach üblichen Verfahren epoxidiert, z. B. mit meta-Chlorperbenzoesäure, zum Diol öffnet und dann mit einem literaturüblichen Phosphorylierungsmittel, wie Phosphoroxychlorid umsetzt.

Bisulfitaddukte der Bis(formylphenyl)ethan-Verbindungen sind durch Reaktion mit Natriumbisulfit leicht zugänglich. Sie können entweder gesondert hergestellt werden oder ohne besondere Isolierung im Test durch Lösen des Dialdehyds in Bisulfitlauge in Situ erzeugt werden.

Die für das erfindungsgemäße Verfahren geeigneten Verbindungen der allgemeinen Formel I werden in mindestens äquivalenter Menge, bezogen auf den Hämoglobingehalt, eingesetzt. Im allgemeinen ist es jedoch von Vorteil, diese Substanzen im Überschuß, vorzugsweise in 2- bis 200fachem, besonders bevorzugt 5 bis 50fachem molarem Überschuß zuzugeben.

Gegebenenfalls ist es zweckmäßig, jedoch nicht unbedingt notwendig, bei Durchführung des erfindungsgemäßen Verfahrens der Meßlösung vor dem Kontaktieren mit Haptoglobin ein Ionenbindungen stabilisierendes Mittel zuzusetzen. Solche Substanzen mit einer stabilisierenden Wirkung auf ionische Bindungen sind beispielsweise Ethylenglykole, Polyethylenglykole oder Saccharose.

Haptoglobin muß ebenfalls in mindestens äquivalenter Menge, bezogen auf das Hämoglobin, eingesetzt werden. Vorteilhaft wirkt sich jedoch auch hier ein Überschuß aus. Vorzugsweise wird die 1,2 bis 50fache, besonders bevorzugt die 1,5 bis 5fache molare Menge verwendet.

Es ist ferner möglich, Haptoglobin auf einen Träger zu fixieren. In diesem Fall kann das Bestimmungsverfahren durchgeführt werden, indem man entweder

a) den mit dem Haptoglobin versehenen Träger in die hämolysierte, gegebenenfalls vorbehandelte Blutprobe eintaucht oder

b) eine definierte Menge der vorbehandelten Blutprobe auf den Haptoglobinträger tropft oder

c) mit einer definierten Menge der vorbehandelten Blutprobe das Haptoglobin vom Träger eluiert.

Als Trägermaterialien eignen sich die für analytische Nachweisreagenzien üblichen Träger, wie Papier, Cellulose, Faservliese, poröse Kunststoffe und dergleichen. Die Herstellung von mit Haptoglobin beladenen Trägern erfolgt durch Eintauchen in oder Besprühen mit einer haptoglobinhaltigen Lösung.

Das glykosilierte Hämoglobin kann auch aufgrund seiner Fähigkeit zur Inhibition der Haptoglobin-induzierten Agglutination von hämoglobinbeladenen Latexpartikeln bestimmt werden. Derartige Latexagglutinationstests sind dem Fachmann als Mittel zur besseren Erkennung der Bildung von Immunkomplexen bekannt und können überraschenderweise auch zur quantitativen Messung der Bindung von glykosiliertem Hämoglobin an Haptoglobin eingesetzt werden.

Das Prinzip der Messung beruht darauf, daß hämoglobinbeladene Latexpartikel in Anwesenheit von Haptoglobin aggregieren und die Lösung trüben. Bei gleichzeitiger Anwesenheit von glykosiliertem Hämoglobin, reagiert Haptoglobin jedoch bevorzugt mit diesem und es findet eine verringerte Agglutination statt. Aus der Geschwindigkeit und dem Ausmaß der Agglutination bzw. der gemessenen Trübung kann mit Hilfe einer Eichkurve der Gehalt an glykosiliertem Hämoglobin in einer Probe ermittelt werden.

Es ist auch folgende Vorgehensweise zur Bestimmung des glykosilierten Anteils möglich, indem die Agglutination von haptoglobinbeladenem Latex durch glykosiliertes Hämoglobin in Gegenwart eines Antikörpers gegen Hämoglobin gemessen wird. Hierfür können poly- oder monoklonale Antikörper gegen glykosiliertes oder nichtglykosiliertes Hämoglobin oder gegen beliebige Mischungen der beiden Formen verwendet werden. In einer bevorzugten Ausführungsform wird ein monoklonaler Antikörper gegen den glykosilierten Aminoterminus der ß-Kette des glykosilierten Hämoglobins zugesetzt, wie er z. B. in dem US-Patent 4,478,744 beschrieben ist.

Bevorzugt ist in jedem Fall der Einsatz von Antikörpern in einer Konzentration von $10^{-4}$ bis $10^{-8}$ mol/l. Die bevorzugte Größe der Latexpartikel liegt zwischen 10 und 300 nm. Die Partikelkonzentration weiß der Fachmann so zu wählen, daß im bevorzugten Wellenbereich zwischen etwa 400 und etwa 1000 nm eine ausreichend empfindliche photometrische Messung der Latexagglutination möglich wird.

Ein wesentlicher Vorteil der Messung der Komplexbildung von Haptoglobin und glykosiliertem Hämoglobin durch Latexagglutination ist im Vergleich zur Detektion anhand der Peroxidaseaktivität die Einsparung eines Inkubationsschrittes, so daß bereits ohne Einsatz der erfindungsgemäßen Substanzen ein vollautomatisierbares Verfahren bestehend aus zwei Inkubationsschritten realisiert werden kann. Besondere Vorteile bietet die kombinierte Anwendung der oben genannten Substanzen der allgemeinen Formel I, welche durch nichtglykosiliertes Hämoglobin hervorgerufene Meßsignale unterbinden oder zumindest deutlich vermindern, und der Detektion durch Latexagglutination. Hierdurch wird ein einfaches, im günstigsten Fall nur aus einem Inkubationsschritt bestehendes Verfahren von hoher Spezifität ermöglicht.

Gegenstand der Erfindung ist ferner ein Reagenz zur automatisierbaren Bestimmung von glykosiliertem Hämoglobin in Blutproben, enthaltend ein Mittel zur Hämolyse der Erythrozyten in einem geeigneten Puffersystem, freies oder trägergebundenes Haptoglobin, eine oder mehrere Verbindungen der allgemeinen Formel I sowie gegebenenfalls Substanzen mit stabilisierender Wirkung auf ionische Bindungen.

Zur Herstellung des Reagenzes in Form eines Teststreifens wird ein saugfähiger Träger, vorzugsweise Papier, Cellulose, Faservlies, poröser Kunststoff oder dergleichen mit Lösungen der erforderlichen Reagenzien in geeigneten Lösungsmitteln imprägniert. Dies kann in einem Imprägnierschritt erfolgen. Oft ist es jedoch zweckmäßig, die Imprägnierung in mehreren Schritten durchzuführen, wobei Lösungen eingesetzt werden, die jeweils einen Teil der Bestandteile des Reagenzes enthalten. Die fertigen Teststreifen können als solche verwendet werden oder in an sich bekannter Weise an Griffe angeklebt oder vorzugsweise zwischen Kunststoffen und feinmaschigen Netzwerken gemäß DBP 21 18 455 eingesiegelt werden.

Ein weiterer Gegenstand der Erfindung ist ein Reagenz zur Differenzierung von glykosiliertem und nichtglykosiliertem Hämoglobin, enthaltend Haptoglobin und hämoglobinbeladene Latexpartikel sowie eine oder mehrere Verbindungen der allgemeinen Formel I.

Ein Reagenz zur Differenzierung von glykosiliertem und nichtglykosiliertem Hämoglobin enthaltend haptoglobinbeladene Latexpartikel, poly- oder monoklonale Antikörper gegen glykosiliertes oder nichtglykosiliertes Hämoglobin sowie eine oder mehrere Verbindungen der allgemeinen Formel I ist ebenfalls Gegenstand der Erfindung.

Die folgenden Beispiele erläutern die Erfindung.

Beispiel 1

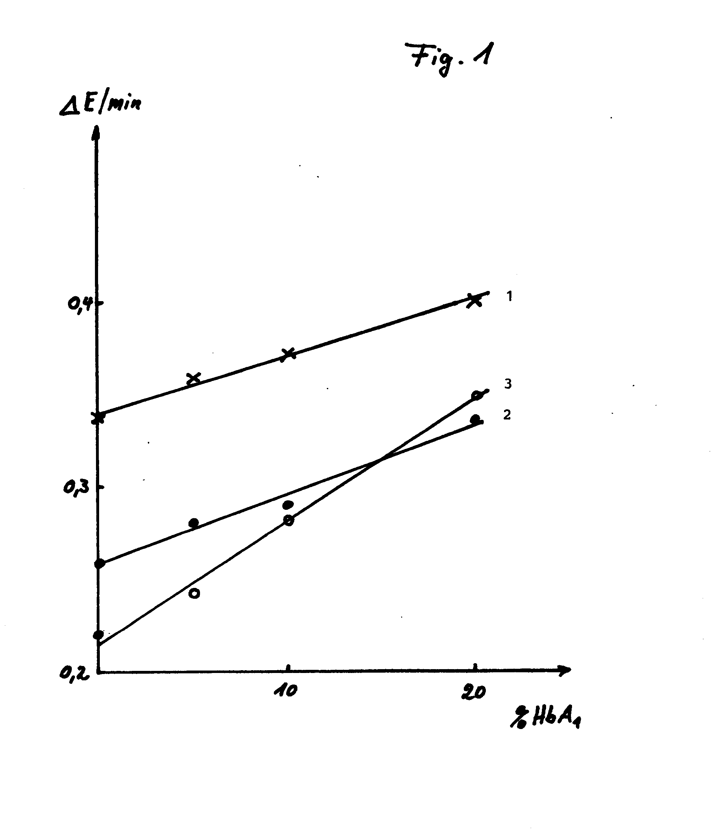

Reines Hämoglobin und glykosiliertes Hämoglobin werden durch Ionenaustauschchromatographie (nach Trivelli et al. NEJM 284, 353-357 (1971) isoliert und zur Herstellung einer Mischungsreihe von 0 - 50 % glykosiliertem Hämoglobin verwendet. Die so erhaltenen Proben werden in parallelen Versuchen mit Lösungen der unten aufgeführten Substanzen in 50 mmol/l Morpholinethansulfonsäure-Puffer pH 6,5 auf eine Hämoglobinkonzentration von jeweils 0,5 mg/l verdünnt. Dann wird festes Natriumdithionit bis zur Konzentration 5 mg/ml und festes Natriumnitrit bis zur Konzentration 0,05 mg/ml zugesetzt und gelöst. Nach 30 min Inkubation bei 25° C wird die so erhaltene Lösung in die Probengefäße eines automatischen Analysengerätes, z. B. Abott VP eingefüllt. Das Gerät mischt jeweils 2,5 µl der Probelösung mit 250 µl einer Reagenzlösung, welche 10 mmol/l Bis-Tris-Puffer pH 6,5, 2 mmol/l 2,2'-Azino-di[3-ethylbenzthiazolin--sulfonat(6)], 0,05 mg/ml Haptoglobin und 11 Vol.-% Äthylenglykol enthält. Es wird 80 sec bei 25° C inkubiert. Danach wird die Pseudoperoxidaseaktivität durch Zugabe von 2,2 mg/ml Natriumperborat in einem 3 g/l Saponin enthaltenden Citratpuffer (0,5 mol/l, pH 3,8) gestartet und mit einer geeigneten Filterkombination, z. B. 415/450 nm, gemessen. Es werden die in Fig. 1 wiedergegebenen Eichgeraden erhalten, wobei

Gerade 1 mit 10 mmol/l

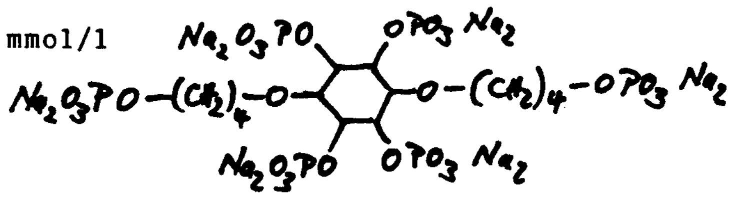

Gerade 2 mit 2 mmol/l

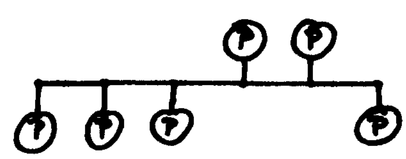

Gerade 3 mit 1 mmol/l

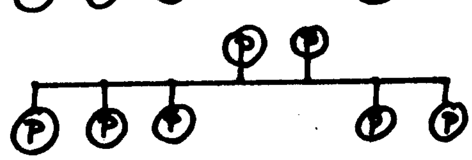

erhalten worden sind (P) = $OPO_3Na_2$).

Beispiel 2

20 µl Vollblut werden in 5 ml einer Lösung hämolysiert, welche 50 mmol/l Bis-Tris-Puffer pH 6,7, 0,2 g/l Saponin und variable Mengen einer der unter a) bis e) aufgeführten Substanzen enthält. Nach einer 20minütigen Inkubation bei 25° C wird ein Aliquot von 20 µl entnommen und mit 2 ml einer Lösung versetzt, welche 7 mmol/l Bis-Tris-Puffer pH 6,7, 2 mmol/l 2,2'-Azino-di-[3-ethylbenzthiazolin-sulfonat(6)] (ABTS), 20 mg/l Haptoglobin und 200 g/l Saccharose enthält. Nach genau zwei weiteren Minuten bei 25° C wird die kinetische Bestimmung der Peroxidaseaktivität gestartet, indem 1 ml einer Lösung aus 0,5 mol/l Citratpuffer pH 3,8, 30 mmol/l Natriumperborat und 3 g/l Saponin zugegeben wird. Aus der gemessenen Geschwindigkeit wird mittels einer Eichgeraden, welche durch Messung von verschiedenen Mischungen reinen glykosilierten und nichtglykosilierten Hämoglobins zu erstellen ist, der Gehalt an glykosiliertem Hämoglobin der Humanblutproben ermittelt.

Die folgende Tabelle zeigt den nach folgenden Varianten bestimmten glykosilierten Hämoglobingehalt verschiedener Proben im Vergleich zu den Werten, welche mittels der gängigen Referenzmethode (Ionenaustauschchromatographie) erhalten wurden.

% glykosiliertes Hämoglobin

| Probe Nr. | 1 | 2 | 3 | 4 |
|---|---|---|---|---|
| Referenzmethode | 6,1 | 12,4 | 8,8 | 13,7 |
| Variante a | 6,9 | 12,6 | 8,4 | 13,5 |
| Variante b | 7,1 | 11,5 | 8,9 | 13,3 |
| Variante c | 6,3 | 12,0 | 9,2 | 12,8 |
| Variante d | 5,8 | 12,6 | 8,3 | 14,0 |
| Variante e | 6,0 | 12,8 | 9,1 | 14,2 |

a) Das Hämolysereagenz enthält 0,1 mmol/l

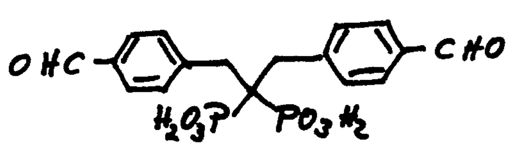

b) Das Hämolysereagenz enthält 0,2 mmol/l

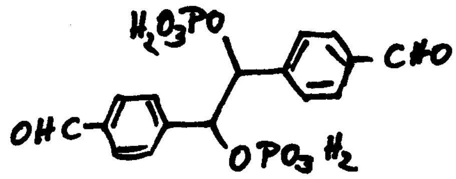

c) Das Hämolysereagenz enthält 0,1 mmol/l

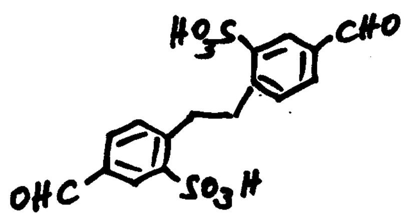

d) Das Hämolysereagenz enthält 2 mmol/l

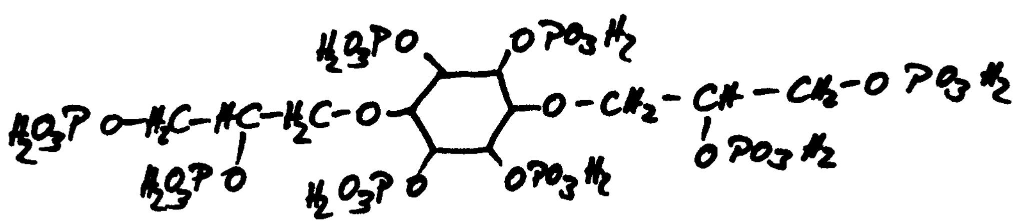

e) Das Hämolysereagenz enthält 0,5 mmol/l

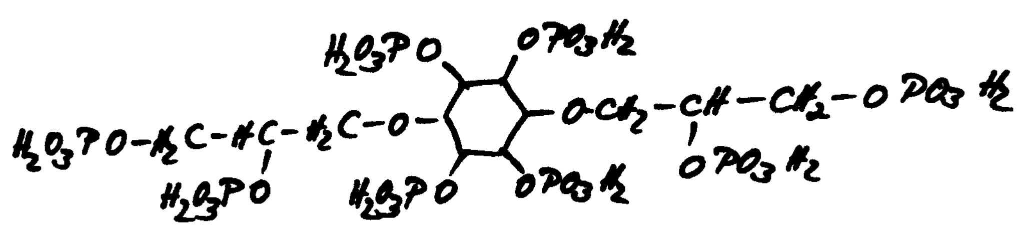

zusätzlich 0,05 g/l Natriumnitrit und 5 g/l Natriumdithionit

Es wird eine durchweg gute Übereinstimmung zwischen den jeweiligen Ergebnissen der verschiedenen Verfahrensvarianten a) bis e) mit denjenigen der Referenzmethode beobachtet.

Beispiel 3

5 ml einer 2 %igen Suspension von handelsüblichen, aktive Epoxidgruppen tragenden Latexpartikeln der durchschnittlichen Größe 160 nm wurden mit 5 ml einer Lösung von 10 mg/ml gereinigtem Oxyhämoglobin $A_1$, 50 mmol/l Na-Phosphatpuffer pH 7,8 und 0,5 mol/l NaCl 3 Stunden unter leichtem Rühren bei Raumtemperatur inkubiert. Nach Zugabe von 10 ml einer 5 %igen Lösung von Rinderserumalbumin wurde weitere 15 Stunden inkubiert. Dann wurde bei 50.000 g abzentrifugiert und zweimal mit 10 mmol/l Bis-Tris-Puffer pH 6,7 gewaschen. Mit diesem Puffer wurde schließlich so stark verdünnt, daß eine Suspension erhalten wurde, welche bei 700 nm eine Extinktion von 0,4 hatte. Zu 2 ml einer derartigen Suspension wurden 100 $\mu$l eines Hämolysats von 20 $\mu$l Vollblut in 5 ml einer Lösung von 50 mmol/l Bis-Tris-Puffer pH 6,7, 0,2 g/l Saponin und 1 mmol/l der Substanz

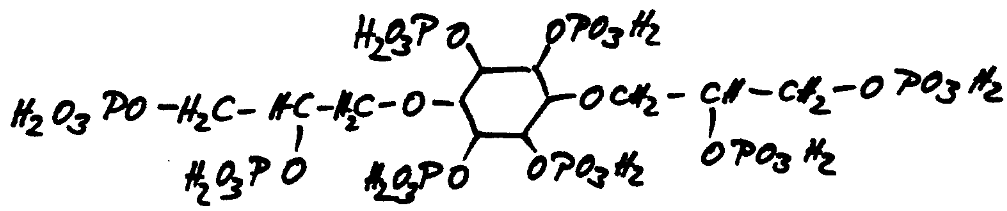

gegeben. Nach gründlichem Durchmischen wurden 20 $\mu$l einer Lösung von 1 g/l Haptoglobin zugegeben. Die Extinktion bei 700 nm wurde nach 30 und 150 sec gemessen. Aus der Extinktionsdifferenz zwischen der ersten und der zweiten Messung wird anhand einer Eichkurve (Mischungsreihe aus glykosiliertem und nichtglykosiliertem Hämoglobin) der Gehalt an glykosiliertem Hämoglobin ermittelt und mit den durch Ionenaustausch-Chromatographie ermittelten Werten verglichen.

% glykosiliertes Hämoglobin

| Probe Nr. | 1 | 2 | 3 | 4 |
|---|---|---|---|---|
| Referenzmethode | 5,9 | 8,3 | 13,1 | 15,3 |
| Latextest | 6,4 | 8,5 | 12,6 | 16,8 |

Die Übereinstimmung der Ergebnisse beider Verfahren ist sehr gut.

Beispiel 4

Analog der Vorgehensweise in Beispiel 3 wird eine Suspension von mit Pferde-Haptoglobin beladenen Latexpartikeln hergestellt. Die erhaltene Suspension wird auf eine Extinktion von 0,4 bei 630 nm verdünnt und enthält außerdem einen der im folgenden aufgezählten anti-Hämoglobin-Antikörper als reines IgG:

a) 0,04 mg/ml eines polyklonalen Antikörpers gegen Humanhämoglobin;

b) 0,1 mg/ml eines polyklonalen Antikörpers gegen die ß-Kette des glykosilierten Humanhämoglobins;

c) 0,5 mg/ml eines monoklonalen Antikörpers gegen den aminoterminalen Teil der ß-Kette von glykosiliertem Humanhämoglobin.

Außerdem enthält die Suspension 0,2 mmol/l der Substanz

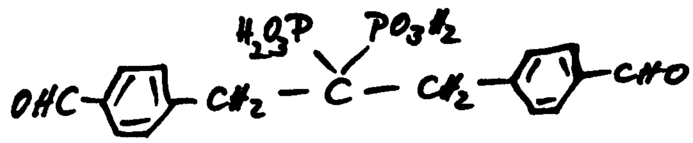

Zu 2 ml einer derartigen auf 25° C temperierten Suspension werden 50 µl eines Hämolysats von 20 µl Vollblut in 5 ml einer Lösung von 50 mmol/l Bis-Tris-Puffer pH 6,7 und 0,2 g/l Saponin zugemischt. Die Extinktionszunahme innerhalb 120 sec bei 630 nm wird gemessen. Der Gehalt an glykosiliertem Hämoglobin wird über eine Eichkurve ermittelt. Die folgende Tabelle zeigt für einige Humanserumproben den Vergleich mit den durch Ionenaustausch-Chromatographie ermittelten Werten

% glykosiliertes Hämoglobin

| Probe Nr. | 1 | 2 | 3 | 4 |
|---|---|---|---|---|
| Referenzmethode | 6,5 | 9,8 | 10,9 | 13,6 |
| Latextest mit | | | | |
| Antikörper a | 7,1 | 8,4 | 12,0 | 14,3 |
| Antikörper b | 6,9 | 8,7 | 11,2 | 13,1 |
| Antikörper c | 6,3 | 10,2 | 11,5 | 14,0 |

Ergebnisse aus der Referenzmethode und den Varianten des Latexagglutinationstests 4 a) - 4 c) zeigen auch hier sehr gute Übereinstimmung.

Beispiel 5

Perseitolheptaphosphat

Eine Lösung von Perseitol (0,5 g, 2.4 mMol) in Polyphosphorsäure (8 g) wird 6 Stunden auf 150° C erwärmt, abgekühlt und in 80 ml Wasser eingetragen. Nach Zugabe von 20 ml 10 N Salzsäure wird diese Lösung 30 Minuten auf 100° C erhitzt, abgekühlt, durch Zugabe von Ammoniak alkalisch gestellt und auf ca. 20 ml eingeengt. Durch Zugabe von konzentrierter wässriger Ammoniaklösung (ca. 120 ml) und Aufbewahren bei 5° C wird anorganisches Phosphat ausgefällt. Das Filtrat wird wiederum auf ca. 20 ml eingeengt, mit Ammoniak auf pH 8 eingestellt und mit gesättigter wässriger Bariumacetat-Lösung versetzt. Zur vollständigen Ausfällung der Titelverbindung als Barium-Salz wird mit Ethanol versetzt, der Niederschlag abgesaugt und getrocknet. Man erhält

1.5 g Perseitolheptaphosphat Ba-Salz als weißes, in Wasser schwerlösliches Pulver mit

Fp $>$ 350° C
$M_{rel}$ 1.2 (0.03 M Oxalat-Puffer, pH 4)
Ausbeute: 36 % der Theorie

## Beispiel 6

## 3,6-Bis-0-(4-hydroxybutyl)-myo-inosithexaphosphat

Eine Mischung des Polyols, 3,6-Bis-0-(4-hydroxybutyl)-myo-inosit (0,5 g, 1.5 mMol), N-Benzoylphosphoramidinsäure (7.5 g, 37.3 mMol) und Triethylamin (5.2 ml, 37.3 mMol) in 50 ml Dimethylformamid wird 24 Stunden auf 140° C erhitzt. Nach Abkühlen und Zugabe von 10 ml Wasser wird zur Trockne eingedampft. Der Rückstand wird mit 25 ml Wasser aufgenommen und zur Entferung des Benzamids mehrmals mit Ether extrahiert. Nach Zugabe von 6 ml 6 N Salzsäure zur wässrigen Lösung wird 30 Minuten auf 100° C erhitzt und nach dem Abkühlen mit wässriger Ammoniak-Lösung versetzt. Die weitere Aufarbeitung erfolgt wie in Beispiel 5 beschrieben. Man erhält

1.2 g 3,6-Bis-0-(4-hydroxybutyl)-myo-inosithexaphosphat
Barium-Salz als
farbloses, pulverförmiges Material mit
Fp $>$350° C
$M_{rel}$ 1.4 (0.03 M Oxalat-Puffer, pH 4)
Ausbeute: 50 % der Theorie

Die als Ausgangsmaterial für vorstehendes Beispiel 6 eingesetzte Verbindung wird folgendermaßen erhalten:

1,2 : 4,5-Bis-0-cyclohexyliden-myo-inosit (14 g, 40 mMol) in 400 ml absolutem Dimethylformamid wird mit einem Überschuß Natriumhydrid (70 %ig; 6.86 g, 200 mMol) bei 0° C versetzt. Nach Beendigung der Wasserstoff-Entwicklung (ca. 10 Minuten) wird Benzyloxybutylbromid (Kp 148 - 150° C, 0.3 mm; 97 g, 400 mMol) zugegeben und 20 Stunden bei 25° C gerührt. Danach wird der Ansatz auf Eis/Wasser gegeben und mehrfach mit Essigester extrahiert. Die organische Phase wird getrocknet, eingeengt und der Rückstand säulenchromatographisch an Kieselgel mit Methylenchlorid/Methanol = 98 : 2 (v/v) gereinigt. Einengen der entsprechenden Fraktion (Rf 0.5) ergibt

19.9 g 3,6-Bis-0-(4-benzyloxybutyl)-1,2:4,5-bis-0-cyclohexyliden-myo-inosit als
farbloses Öl
Ausbeute: 84 % der Theorie

Katalytische Hydrierung (10 % Pd/C; 40° C, 50 bar Wasserstoff-Atmosphäre, 6 Stunden) der so erhaltenen Verbindung (20 g, 34 mMol) in 2 l Ethanol unter Zusatz von konzentrierter wässriger Ammoniak-Lösung (5 ml) ergibt

7 g 1,2:4,5-Bis-0-cyclohexyliden-3,6-bis-0-(4-hydroxybutyl)-myo-inosit
Fp 108 - 110° C
Ausbeute: 44 % der Theorie

Saure Hydrolyse (50 ml 0.4 %ige Schwefelsäure in 50 ml 1,4-Dioxan, 100° C, 2 Stunden) dieser Biscyclohexylidenverbindung (5 g, 10 mMol) ergibt nach Neutralisation mit Natriumhydrogencarbonat, Entfernung des Lösungsmittels, Extraktion des Rückstandes mit Methanol und Konzentration der Extraktionslösung

3 g 3,6-Bis-0-(4-hydroxybutyl)-myo-inosit
Fp 74 - 76° C
Ausbeute: 93 % der Theorie

Beispiel 7

5-Amino-1-hydroxypentan-1,1-diphosphonsäure

In 60 ml Chlorbenzol gibt man 14 g 5-Aminovaleriansäure sowie 13,5 g phosphorige Säure und erhitzt 10 Minuten auf 100° C. Nach dem Entfernen des Ölbades tropft man langsam 15,7 ml Phosphortrichlorid zu und erhitzt weitere 3 Stunden auf 100° C. Nach dem Abkühlen wird das Chlorbenzol abdekantiert, der Rückstand mit 6 normaler Salzsäure 5 Stunden unter Rückfluß erhitzt und anschließend im Vakuum eingeengt. Das zurückbleibende gelbe Öl löst man in 100 ml Wasser, stellt mit 2 N Natronlauge auf pH 5 und versetzt mit 200 ml Methanol. Der Niederschlag wird abgesaugt und im Vakuum getrocknet. Man erhält 24,5 g (67 % der Theorie) der gewünschten Verbindung als Mononatriumsalz mit einer relativen Mobilität von 0,45.

Durch Ansäuern einer wässrigen Lösung des Natriumsalzes mit 6 normaler Salzsäure erhält man die aus Wasser umkristallisierbare freie Diphosphonsäure mit Fp 240 - 245° C (Zersetzung).

## Beispiel 8

### 6-Amino-1-hydroxyhexan-1,1-diphosphonsäure

Analog zu dem in Beispiel 7 beschriebenen Verfahren erhält man ausgehend von 6-Amino-hexansäure 6-Amino-1-hydroxyhexan-1,1-diphosphonsäure in 43 %iger Ausbeute mit einer relativen Mobilität von 0,42 und Fp: 218 - 221° C (Zersetzung).

## Beispiel 9

### 3,6-Bis-O-(4-oxobutyl)-myo-inosit

Eine Lösung von 1,2 : 4,5-Bis-O-cyclohexyliden-3,6-bis-O-(4-oxobutyl)-myo-inosit (1,5 g, 3,1 mMol) in 30 ml Ethanol, 6 ml Wasser und 1 ml konzentrierter Salzsäure wird 2 Stunden auf 60° C erwärmt. Nach dem Abdampfen des Lösungsmittels wird über Kieselgel chromatographiert, zuerst mit Methylenchlorid zur Entfernung einer schnell-laufenden Verunreinigung und schließlich mit Methylenchlorid/Methanol = 4 : 1 (v/v). Einengen der entsprechenden Fraktion (Rf 0.08) ergibt

0,3 g 3,6-Bis-O-(4-oxobutyl)-myo-inosit als
farbloses Öl
Ausbeute: 30 % der Theorie

Das eingesetzte Ausgangsmaterial wird erhalten durch Oxidation von 1,2 : 4,5-Bis-O-cyclohexyliden-3,6-bis-O-(4-hydroxybutyl)-myo-inosit (2 g, 4,1 mMol) mit Bariummanganat (30 g, 117 mMol) in 100 ml Toluol bei Siedetemperatur während 12 Stunden. Nach Abfiltrieren des Niederschlags und Einengen des Filtrats wird säulenchromatographisch über Kieselgel mit Toluol/Essigester = 1:1 (v/v) gereinigt. Einengen der Fraktion mit Rf 0.4 ergibt

0.8 g 1,2:4,5-Bis-O-cyclohexyliden-3,6-bis-O-(4-oxobutyl)-myo-inosit
farbloses Öl
Ausbeute: 41 % der Theorie

Beispiel 10

3,6-Bis-0-(4-oxobutyl)-myo-inosittetraphosphat

Eine Mischung von 3,6-Bis-0-(4-oxobutyl)-myo-inosit (0.6 g, 1.87 mMol) aus Beispiel 9, N-Benzoylphosphoramidinsäure (7.23 g, 36 mMol) und Triethylamin (5 ml, 36 mMol) in 50 ml Dimethylformamid wird 24 Stunden auf 140° C erhitzt. Nach Abkühlen und Zugabe von 10 ml Wasser wird zur Trockne eingedampft. Der Rückstand wird mit 25 ml Wasser aufgenommen und zur Entfernung des Benzamids mehrmals mit Ether extrahiert. Nach Zugabe von 6 ml 6 N Salzsäure zur wässrigen Lösung wird 30 Minuten auf 100° C erhitzt und nach dem Abkühlen wird durch Zugabe von Ammoniak alkalisch gestellt und auf ca. 20 ml eingeengt. Durch Versetzen mit 120 ml konzentrierter wässriger Ammoniak-Lösung und Aufbewahren bei 5° C wird anorganisches Phosphat ausgefällt. Das Filtrat wird wiederum auf ca. 20 ml eingeengt, mit Ammoniak auf pH 8 eingestellt und mit gesättigter wässriger Bariumacetat-Lösung versetzt. Zur vollständigen Ausfällung der Titelverbindung als Bariumsalz wird mit etwas Ethanol versetzt. Der Niederschlag wird abgesaugt (1.05 g, 0.89 mMol) und in 50 ml Wasser suspendiert, mit Kationenaustauscher Amberlite IR 120 ($H^+$-Form) (5 ml) während 30 Minuten bei 25° C behandelt. Nach Abfiltrieren des Ionenaustauscher-Harzes wird das Filtrat mit 18 ml 0.1 M Natrium-methylatlösung versetzt und eingeengt. Man erhält

0.57 g 3,6-Bis-0-(4-oxobutyl)-myo-inosittetraphosphat Natrium-Salz
als weißes, in Wasser gut lösliches Pulver
Fp $>$ 350° C
$M_{rel}$ 1,3 (0,03 M Oxalat-Puffer, pH 4)
Ausbeute: 37 % der Theorie

Beispiel 11

1,2-Bis(4-formyl-2-sulfonylphenyl)ethan

a) 4-Brommethylbenzoesäureethylester

50 g 4-Methylbenzoesäureethylester werden mit 60 g N-Bromsuccinimid und 3 Spatelspitzen Dibenzoylperoxid 3 Stunden in 100 ml Tetrachlorkohlenstoff unter Rückfluß erhitzt. Man rührt noch 12 Stunden bei Raumtemperatur, saugt dann ab, wäscht den Niederschlag mit Tetrachlorkohlenstoff und dampft das Filtrat ein. Der ölige Rückstand wird bei 0,3 mbar destilliert. Die Fraktion bei 105° C wird aus 50 ml n-Hexan kristallisiert.

Ausbeute: 52,3 g
Schmelzpunkt: 24 - 29° C

b) Triphenyl-(4-ethoxycarbonylbenzyl)phosphoniumbromid

50 g der Brommethylverbindung aus Beispiel 11 a) und 54 g Triphenylphosphin werden 3 Stunden in 200 ml Toluol unter Rückfluß erhitzt. Man saugt das Produkt ab, wäscht mit Toluol nach und trocknet.

Ausbeute 92,8 g
Schmelzpunkt: 233 - 236° C

c) 4-Ethoxycarbonyl-4'-methoxycarbonylstilben

In 150 ml trockenem 1,4-Dioxan werden 43,5 g des oben beschriebenen Phosphoniumbromids und 10,63 g Kalium-tert-butoxid gelöst. Nach 10 Minuten Rühren bei Raumtemperatur gibt man noch 150 ml 1,4-Dioxan zu und tropft dann 14,1 g 4-Formylbenzoesäuremethylester, gelöst in 80 ml 1,4-Dioxan, zu. Man rührt über Nacht bei Raumtemperatur, dampft ein und digeriert mit 150 ml Methanol. Man filtriert das Produkt ab und wäscht noch dreimal mit Methanol.

Ausbeute: 22,2 g

$^1$H-NMR: ([$D_6$]-DMSO):
$\delta$ = 1.34 (t, J = 6.9 Hz, 3 H); 3,86 (s, 3H);
4.32 (q, J = 6,9 Hz, 3 H); 7.45 (s, 2H);
7.74 und 7.94 ppm je d, J = 9 Hz, 8 H)

d) 1-(4-Ethoxycarbonylphenyl)-2-(4-methoxycarbonylphenyl)ethan

20 g des oben beschriebenen Stilbens werden in 400 ml Dimethylformamid und 100 ml Methanol aufgenommen und mit 1g Palladium auf Aktivkohle bei Atmosphärendruck hydriert. Nach 2,5 Stunden ist die theoretische Menge Wasserstoff verbraucht. Man filtriert vom Katalysator ab, dampft ein und kristallisiert aus Methanol.

Ausbeute: 14,4 g
Schmelzpunkt 60 - 62° C

$^1$H-NMR: ([$D_6$]-DMSO):
$\delta$ = 1.31 (t, J = 6.9 Hz, 3 H); 2.99 (s, 4 H); 3.83 (s, 3 H);
4.48 (q, J = 6.9 Hz, 2 H); 7.31 und 7.83 ppm (je"d",
J = 9 Hz, 8 H)

e) 1,2-Bis(4-hydroxymethylphenyl)ethan

10 g 1-(4-Ethoxycarbonylphenyl)-2-(4-methoxycarbonylphenyl)-ethan gelöst in 150 ml trockenem 1,4-Dioxan, werden langsam zu einer Suspension von 3,65 g Lithiumaluminiumhydrid in 200 ml trockenem Ether getropft. Nach 30 Minuten Rühren tropft man 50 ml Essigester langsam zu und rührt 15 Minuten. Danach werden noch 20 ml kalt gesättigte wässrige Natriumsulfatlösung zugetropft. Man gibt zur Bindung des Wassers noch festes Natriumsulfat zu und filtriert. Der Natriumsulfatniederschlag wird noch je dreimal mit 30 ml Essigester ausgerührt und filtriert. Die vereinigten organischen Filtrate werden eingedampft und aus Methanol kristallisiert.

Ausbeute: 5,62 g
Schmelzpunkt: 145 - 149° C

$^1H$-NMR ($[D_6]$-DMSO):
$\delta$ = 2.84 (s, 4 H); 4.43 (d, J = 6 Hz, 4 H);
4.97 (t, J = 6 Hz, 2 H); 7.13 und 7.17 ppm
(je d, J = 8 Hz, 8 H)

f) 1,2-Bis(4-formylphenyl)ethan

4,27 g des in Beispiel 11 e) beschriebenen Bisbenzylalkohols werden 7 Tage bei Raumtemperatur mit 25,5 g Braunstein in 200 ml Petrolether (Kp 60 - 90° C) gerührt. Man filtriert, dampft ein und chromatographiert an Kieselgel (Eluens: Chloroform/Essigester 9:1; Rf-Wert des Produkts an Kieselgel-DC: 0,78).

Ausbeute: 1,47 g

$^1H$-NMR ($[D_6]$-DMSO):
$\delta$ = 3,04 (S, 4 H); 7,44 und 7,80 (je d, J = 8 Hz, 8 H);
9,87 ppm (s, 2 H)

g) Bisulfit-Addukt

200 mg des Dialdehyds aus Beispiel 11 f) werden in 20 ml Ethanol gelöst. Dazu tropft man 4 ml 1 M Natriumbisulfit-Lösung. Der Niederschlag wird filtriert und getrocknet.

Elementaranalyse: $C_{16}H_{14}Na_2S_2O_8$ x $H_2O$
Ber: C 41,6, H 3,5, Na 10,0;
Gef: C 40,2, H 3,49, Na 10,7

h) 1,2-Bis(4-formyl-2-sulfonylphenyl)ethan

Zu 100 ml Chlorsulfonsäure gibt man unter Rühren 10 g 1,2-Bis(4-formylphenyl)-ethan und rührt 2 Stunden bei 85° C. Der Ansatz wird heiß auf Eiswasser gegeben. Der dabei ausgefallene Niederschlag wird abfiltriert und mit Wasser gewaschen. Man suspendiert den Niederschlag in 100 ml 2N HCl und erhitzt 2 Stunden unter Rückfluß. Nach Eindampfen wird das Rohprodukt über Kieselgel (Eluent Butanol/Eisessig/Wasser 4:0,5:1) chromatographiert. Die Fraktionen, die das Produkt enthalten, werden eingedampft und an Diaion (Eluent Wasser) entsalzt.

Ausbeute: 1,5 g
Rf: (Kieselgel-DC, Fließmittel Butanol/Eisessig/Wasser 4:0,5:1) : 0,34

## Patentansprüche

1. Verfahren zur Bestimmung von glykosiliertem Hämoglobin in Blutproben, wobei zunächst durch chemische oder physikalische Behandlung der Blutprobe glykosiliertes und nicht glykosiliertes Hämoglobin aus den Erythrozyten freigesetzt, gegebenenfalls das Hämoglobin in Methämoglobin übergeführt, danach eine Differenzierung von glykosiliertem und nicht glykosiliertem Hämoglobin mittels Haptoglobin durchgeführt und anschließend der glykosilierte Anteil des Hämoglobins bestimmt wird, dadurch gekennzeichnet, daß die Differenzierung von glykosiliertem und nicht glykosiliertem Hämoglobin mit Haptoglobin in Gegenwart eines geeigneten Puffersystems und einer oder mehreren Substanzen der allgemeinen Formel I

$$A\ (BR)_n \qquad (I)$$

in der

A ein gesättigter oder ungesättigter, geradkettiger oder verzweigter aliphatischer Kohlenwasserstoffrest, der ein- oder mehrfach durch Heteroatome, durch Oxycycloalkoxyreste oder durch aromatische Reste unterbrochen sein kann, oder ein aromatischer Rest,

R eine Hydroxy-, Hydroxyalkyl-, Hydroxyalkoxygruppe, eine Aminogruppe, eine reaktive Gruppe X, einen Säurerest bzw. dessen Salze oder einen ein- oder mehrfach durch Säurereste bzw. deren Salze oder durch reaktive Gruppen X substituierten Alkyl-, Alkoxy-, Hydroxyalkyl- oder Hydroxyalkoxyrest, die durch Heteroatome unterbrochen sein können,

B eine Einfachbindung oder einen unsubstituierten oder einen ein- oder mehrfach durch weitere Reste R substituierten aromatischen Rest, und

n eine ganze Zahl von 2 bis 20

bedeuten,

wobei sowohl die verschiedenen Reste B als auch R innerhalb einer Substanz auch unterschiedlich sein können,

erfolgt.

2. Verfahren gemäß Anspruch 1, dadurch gekennzeichnet, daß die Differenzierung von glykosiliertem und nichtglykosiliertem Hämoglobin mittels Haptoglobin in Gegenwart von Substanzen durchgeführt wird, welche ionische Bindungen ausbilden können.

3. Verfahren gemäß Anspruch 2, dadurch gekennzeichnet, daß als Substanzen Verbindungen der allgemeine Formel II

$$A'(R')_n \qquad (II)$$

in der

A' ein gesättigter oder ungesättigter, geradkettiger oder verzweigter aliphatischer Kohlenwasserstoffrest, der ein- oder mehrfach durch Heteroatome oder Oxycycloalkoxyreste unterbrochen sein kann, oder ein aromatischer Rest,

R' eine Hydroxy- oder Aminogruppe, ein Säurerest bzw. dessen Salze, einen ein- oder mehrfach durch Säurereste bzw. deren Salze substituierter Alkyl-, Alkoxy-, Hydroxyalkyl- oder Hydroxyalkoxyrest, wobei die Reste R' innerhalb einer Substanz gleich oder verschieden sein können, und

n eine ganze Zahl von 2 bis 20

bedeuten.

4. Verfahren gemäß Anspruch 1, dadurch gekennzeichnet, daß die Differenzierung von glykosiliertem und nichtglykosiliertem Hämoglobin in Gegenwart von Substanzen durchgeführt wird, welche ionische und kovalente Bindungen ausbilden können.

5. Verfahren gemäß Anspruch 4, dadurch gekennzeichnet, daß als Substanzen Verbindungen der allgemeinen Formel III eingesetzt werden

$$X^1\text{-}B^1\text{-}A''\text{-}B^2\text{-}X^2 \qquad \text{(III)}$$

in der

A''dieselbe Bedeutung wie A in Formel I hat, wobei A'' ein- oder mehrfach durch Reste R substituiert sein können, wobei R die in Formel I angegebene Bedeutung hat,
$B^1$ und $B^2$, die gleich oder verschieden sein können, dieselbe Bedeutung wie B in Formel I haben, und
$X^1$ und $X^2$, die gleich oder verschieden sein können, jeweils eine reaktive Gruppe

vorstellen.

6. Verfahren gemäß Anspruch 5, dadurch gekennzeichnet, daß Verbindungen der allgemeinen Formel IV

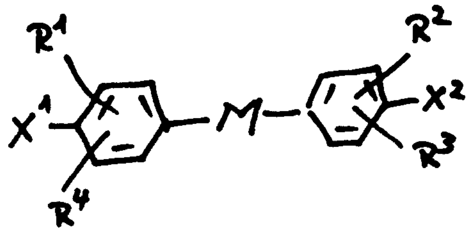

IV

in der

**M** eine Einfachbindung oder eine gesättigte oder ungesättigte Kohlenwasserstoffkette, die gegebenenfalls ein- oder mehrfach durch Säurereste bzw. deren Salze substituiert ist, bedeutet,

$R^1$, $R^2$, $R^3$ und $R^4$, die gleich oder verschieden sein können, Wasserstoff, eine Hydroxygruppe, einen Säurerest bzw. dessen Salze oder einen gegebenenfalls ein- oder mehrfach durch Säurereste bzw. deren Salze substituierter Alkyl-, Alkoxy-, Hydroxyalkyl- oder Hydroxyalkoxyrest, und

$X^1$ und $X^2$, die gleich oder verschieden sein können, eine reaktive Gruppe vorstellen.

7. Verfahren gemäß einem der Ansprüche 1 - 6, dadurch gekennzeichnet, daß die Bestimmung des glykosilierten Anteils des Hämoglobins mittels eines Latexagglutinationstests erfolgt.

8. Reagenz zur Bestimmung von glykosiliertem Hämoglobin in Blutproben, enthaltend ein Mittel zur Hämolyse der Erythrozyten, ein geeignetes Puffersystem, freies oder trägergebundenes Haptoglobin, und gegebenenfalls Substanzen mit stabilisierender Wirkung auf ionische Bindungen, dadurch gekennzeichnet, daß es zusätzlich eine oder mehrere Verbindungen der allgemeinen Formel I enthält.

9. Reagenz zur Differenzierung von glykosiliertem und nichtglykosiliertem Hämoglobin enthaltend Haptoglobin und Hämoglobin-beladene Latexpartikel, dadurch gekennzeichnet, daß es zusätzlich eine oder mehrere Verbindungen der allgemeinen Formel I enthält.

10. Reagenz zur Differenzierung von glykosiliertem und nichtglykosiliertem Hämoglobin, enthaltend Haptoglobin-beladene Latexpartikel und poly- oder monoklonale Antikörper gegen glykosiliertes oder nichtglykosiliertes Hämoglobin, dadurch gekennzeichnet, daß es zusätzlich eine oder mehrere Verbindungen der allgemeinen Formel I enthält.

Fig. 1